# EUROPEAN PATENT SPECIFICATION

(11) **EP 1 725 679 B1**
(45) Date of publication and mention of the grant of the patent: **03.06.2009**
(21) Application number: 05710924.1
(22) Date of filing: 02.03.2005
(51) Int. Cl.: C12Q 1/68

(54) **Treatment monitoring of tumors using AC133 mRNA**
Therapieüberwachung für Tumoren unter Benutzung ACC133 mRNA
Procédé de suivi de thérapie des tumeurs utilisant AC133 mRNA

(30) Priority: 02.03.2004 EP 04075686; 02.03.2004 US 549450 P
(43) Date of publication of application: 29.11.2006
(73) Proprietor: PrimaGen Holding B.V., 1105 BA Amsterdam (NL); UMC Utrecht Holding B.V., 3584 CM Utrecht (NL)
(72) Inventor: PENNING, Maarten, Tjerk, NL-3737 ME Groenekan (NL); VAN DEN BROEK, Sebastiaan, Johannes, Jacobus, NL-1703 MG Heerhugowaard (NL); VOEST, Emile, Eugene, NL-3768 AL Soest (NL); BEEREPOOT, Laurens, Victor, NL-3572 GZ Utrecht (NL); MEHRA, Niven, NL-3532 AH Utrecht (NL)
(74) Representative: van Loon, C.J.J.
(86) International application number: PCT/NL2005/000155
(87) International publication number: WO 2005/083123

(56) References cited:
- EP-A- 1 229 130
- WO-A-02/070735
- WO-A-03/066097
- WO-A-20/04019864
- LIN ET AL: "Real time quantitative RT-PCR of AC133 as surrogate angiogenic marker in colorectal (CRC) patients" PROCEEDINGS OF THE AMERICAN ASSOCIATION FOR CANCER RESEARCH ANNUAL MEETING, vol. 44, July 2003 (2003-07), pages 1071-1072, XP008033635
- YU YING ET AL: "Endothelial progenitor cells in infantile hemangioma." BLOOD, vol. 103, no. 4, 15 February 2004 (2004-02-15), pages 1373-1375, XP002291469 ISSN: 0006-4971
- MAW MARION A ET AL: "A frameshift mutation in prominin (mouse)-like 1 causes human retinal degeneration" HUMAN MOLECULAR GENETICS, vol. 9, no. 1, January 2000 (2000-01), pages 27-34, XP002291470 ISSN: 0964-6906
- MANCUSO PATRIZIA ET AL: "Origin, Kinetics and Viability of Increased Circulating Endothelial Cells in Preclinical Cancer Models and in Cancer Patients." BLOOD, vol. 100, no. 11, 16 November 2002 (2002-11-16), page Abstract No. 983, XP001194800 & 44TH ANNUAL MEETING OF THE AMERICAN SOCIETY OF HEMATOLOGY; PHILADELPHIA, PA, USA; DECEMBER 06-10, 2002 ISSN: 0006-4971
- BALDUS CLAUDIA D ET AL: "High resolution mapping of chromosome 21 genomic dosage and gene expression studies reveal frequent amplification of the 25-30 MB region in 21q21 and overexpression of the APP gene in acute myeloid leukemia (AML) subsets." BLOOD, vol. 102, no. 11, 16 November 2003 (2003-11-16), page 363a, XP001194693 & 45TH ANNUAL MEETING OF THE AMERICAN SOCIETY OF HEMATOLOGY; SAN DIEGO, CA, USA; DECEMBER 06-09, 2003 ISSN: 0006-4971

## Description

The invention relates to the field of medicine. The invention particularly relates to the fields of molecular biology and detection methods.

Recent advances in the knowledge of molecular processes in an organism and techniques to study these processes have resulted in improved methods of typing and treating diseases. Research is being carried out in many fields in order to provide and/or improve methods for diagnosis and treatment of disease as well as providing and/or improving methods for monitoring (side)effects of treatment.
Currently, treatment involving counteracting or enhancing angiogenesis is widely used for a broad spectrum of diseases. Angiogenesis (generation and/or maintenance of blood vessels) often plays an important role in recovery from disease. For instance, active growth of blood vessels is involved with regenerative treatment. Treatment of heart and coronary diseases aims at generation of new blood supply to affected organs by means of new blood vessels.

Angiogenesis is also involved with the onset, and/or development, of many different kinds of diseases such as tumor growth. It is well established that the growth of tumors beyond 1-2mm³ is dependent upon the formation of new blood vessels. On the one hand, blood vessels are required to carry nutrients to the site of the tumor, whereas on the other hand waste material needs to be transported from the tumor. Angiogenesis can be triggered by tumors by secretion of specific chemokines or cytokines. During angiogenesis, endothelial cells proliferate and become motile, moving towards the source of the angiogenic stimulus (e.g. the tumor), degrading the basement membrane and forming primitive vessels. At the same time, the cells increase their proliferative rate from near quiescent to approaching that of bone marrow. This new growth occurs in response to a number of vascular growth factors (Folkman, J. Nature.Med., 1: 27-31, 1995; Miller, Breast Cancer Res.Treat, 2002). In view of this dependency on angiogenesis, tumor treatment often involves anti-angiogenesis drugs.

Because of the common role of angiogenesis during the course of disease and treatment, current methods of diagnosing and staging of disease, and/or methods for monitoring the treatment of disease, are often focused on endothelial cells, since endothelial cells proliferate and become motile during angiogenesis. Mancuso et al determined the number of circulating endothelial cells in cancer patients as compared to healthy controls. They report an increased number of circulating endothelial cells in 76 cancer patients. Recently, it was shown that in cancer patients circulating endothelial cells are increased during progressive disease, and that patients with stable disease had circulating endothelial cell numbers equivalent to healthy volunteers (Beerepoot, L. Ann.Oncol., 15: 139-145, 2004).

Lin et al., 2003 (Proc. Am, Ass. Cancer res. Annual Meeting, vol 44, 1071-1072) discloses the determination of the expression level of AC133 mRNA in peripheral blood samples taken from patients with and without active colorectal cancer (CRC), as well as healthy controls; the study shows a higher AC133 mRNA level in the patients' samples compared to the controls; and a significant higher level of AC133 mRNA in the samples of those patients with active disease status (cf. whole document); the method is based on RT-PCR and GAPDH is used as internal control.

WO2004019864 shows the determination of the expression level of AC133 mRNA in peripheral blood samples taken from patients and controls with colorectal cancer (CRC); the study shows a higher AC133 mRNA level in the patients' samples compared to the controls; and a significant higher level of AC133 mRNA in the samples of those patients with active disease status (cf. Example 2); the method is based on RT-PCR and GAPDH is used as internal control.

Despite much research aiming at developing methods for diagnosis and screening, there remains a need for efficient methods for diagnosis of disease and monitoring of treatment. Diagnosis and monitoring is not always possible or requires complicated, expensive and/or time-consuming procedures which are often inconvenient for a patient, such as obtaining samples, for instance biopsy samples, from a patient and studying these samples in a laboratory. Radiological analysis of tumor cells is only possible weeks after start of tumor therapy.

Described is a method for determining whether an individual is suffering from, or is at risk of suffering from, a disease. It is further provided a method for determining whether a tumor is stable or progressive. Furthermore, it is provided a method for monitoring treatment of an individual suffering from a disease.

The present inventors have demonstrated that the amount of an expression product of AC133 in a sample from an individual is indicative for a disease or for the treatment thereof. It is for instance shown that the expression of AC133 in untreated cancer patients is significantly higher compared to healthy individuals. There is also shown in the examples that AC133 expression significantly drops when various tumor patients are treated, while the total number of circulating endothelial cells remains essentially the same during the same treatment. Hence, according to the present invention, the amount of AC133 expression product is indicative for disease or for the treatment thereof.
The description provides a method for typing a sample of an individual suffering from, or at risk of suffering from, a disease comprising determining whether said sample from said individual comprises an expression product of AC133 in an amount that is indicative for said disease or for the treatment thereof. In one aspect a method is used for diagnosis of disease. People can be routinely tested with a method within certain time intervals. Alternatively, people can be tested when clinical symptoms occur. An unusual amount of AC133 expression product in a sample of said individual, as compared to natural amounts in healthy individuals, is indicative for (a risk of) a certain degree of disease.
The invention furthermore provides a method for monitoring treatment of an individual suffering from a disease comprising determining whether a sample from said individual, undergoing said treatment or having undergone said treatment, comprises an expression product of AC133 in an amount that is indicative for said treatment. As defined herein, typing a sample of an individual means determining whether said sample is indicative for disease or for the treatment thereof. Monitoring treatment of an individual means that therapeutic activity and/or possible side-effects of said treatment are determined, preferably during a certain time interval. Therapeutic activity means the capability of at least in part treating a disease. In one embodiment said therapeutic activity comprises a therapeutic activity against a tumor related disease and/or a blood vessel related disease. A blood vessel related disease is defined herein as a disease involving generation, maintenance and/or breakdown of blood vessels.

AC133, also called CD133, was first described in 1997. It was supposed to be a marker for human hematopoietic stem and progenitor cells (Yin, Blood, 1997; Miraglia, Blood, 1997). Most of the CD34+VEGF-R2+ endothelial cells express AC133. Mature endothelial cells do not express AC133.
Recently, a second isoform of AC133 with a 26 nucleotide deletion was described. This isoform, called AC133-2, is the isoform that is expressed on hematopoietic stem cells. The surface antigen that is recognized by anti-AC133 monoclonal antibodies in the art that are used for the isolation of hematopoietic stem cells recognize AC133-2 and not AC133-1 (Yu, J.Biol.Chem, 2002).
The length of the AC133 mRNA is 3794 nucleotides. By comparison of the mRNA sequence (GenBank accession: AF027208) to a sequence of 115812 nucleotides of genomic homo sapiens chromosome 4 (GenBank accession: NT_006344), the total coding sequence of AC-133 turns out to comprise 27 exons.

An individual is defined herein as an animal comprising blood vessels. Preferably, said animal is a mammal. In one preferred embodiment, said individual is a human individual.

In a method it is determined whether a sample comprises an expression product of AC133 in an amount that is indicative for disease or for treatment thereof. According to the invention, the amount of AC133 expression product is correlated to the status of an individual. A diseased individual has an altered AC133 expression as compared to a healthy individual. Moreover, treatment of a disease can be monitored by determining the amount of AC133 expression product, preferably at several time points. The amount of AC133 expression product is determined using any method known in the art. The art provides various methods for determining the amount of AC133 expression product. In one embodiment, said AC133 expression product comprises protein. The amount of protein is for instance determined using (capture) ELISA, Western blotting, a biosensor, etcetera. In one embodiment the amount of protein is compared with a reference value, preferably the amount of protein present in a comparable sample of the same individual before the start of therapy. Alternatively, mean values of comparable samples of a healthy and/or diseased population are used as a reference.
In a preferred embodiment said AC133 expression product comprises RNA. More preferably, said RNA comprises mRNA because mRNA has a short half-life and the amount of mRNA therefore more accurately reflects the actual status of AC133 expression. In order to detect RNA, an amplification reaction such as a NASBA amplification reaction is often preferred. Specific amplification of a target nucleic acid sequence is achieved by adding two primer sequences to an amplification reaction mixture. The original amount of RNA can be determined in various ways. An amplified region is for instance detected at the end of an amplification reaction by probes that are specific for said amplified region. Alternatively, an amplified region is detected during generation of said amplified nucleic acid in said amplification reaction³. In the latter protocol a signal of a label attached to a probe becomes detectable after said probe has hybridised to a complementary nucleic acid. Examples of such probes that enable real-time homogenous detection in amplification reactions are TaqMan³ and Molecular Beacon probes^{4;5}.
Preferably, the amount of AC133 RNA is compared with a reference value, for instance the amount of AC133 RNA present in a comparable sample of the same individual before the start of therapy. Alternatively, mean values of comparable samples of a healthy and diseased population are used as a reference.
By a comparable sample is meant the same kind of sample. Hence, if the amount of AC133 expression product in a blood sample is measured after start of therapy, a "comparable sample" means another blood sample, preferably taken before the start of therapy. Preferably, said sample obtained after start of therapy and said comparable sample are similarly processed. In one aspect of the invention the same kind of nucleic acid amplification reaction is performed with both samples.
In one embodiment, samples which are compared with each other, for instance samples that are taken before and after treatment, contain essentially the same volumes (liquid samples) or sizes (tissue samples). Alternatively, said samples contain different volumes/sizes. In that case, the difference between the volumes/sizes of said samples are taken into account when comparing the amount of expression product. For instance, if a blood sample taken after start of therapy contains only half of the volume of a sample taken before treatment, the measured amount of AC133 in said sample taken after start of therapy should be multiplied by two in order to allow a direct comparison with the amount of AC133 expression product in said sample taken before treatment. Of course, it is also possible in that case to divide the amount of AC133 expression product present in said sample taken before treatment by two in order to directly compare it with the amount of AC133 expression product present in said sample taken after start of treatment.

A change in the amount of expression product of AC133 is indicative for whether a treatment is effective or not. In one embodiment this change in the amount of AC133 expression product is due to an altered expression by cells involved with said disease, for instance by tumor cells and/or surrounding tissue. In one embodiment however, AC133 expression of other cells that are not directly involved with disease, such as cells in blood circulation, is determined.

In one embodiment, the amount of AC133 expression product is reduced when a treatment is effective. In this case, it is determined whether a treatment is effective by determining whether the amount of AC133 expression product reduces over time. To enable more accurate comparisons with a reference value, the amount of AC133 expression product is preferably quantified. Known methods in the art are suitable for this purpose. Quantification of a target nucleic acid sequence is commonly accomplished by adding a competitor molecule, which is amplified using the same primers and which contains sequences that allow discrimination between competitor and target nucleic acid sequence^{2;6}. The ratio between amplified competitor and target nucleic acid sequence is used to quantify said target nucleic acid sequence. Detection of competitor or target nucleic acid sequence is for instance achieved at the end of the amplification reaction by probes that are specific for said amplified region of competitor or target nucleic acid sequence or during generation of said amplified nucleic acid in the amplification reaction. In the latter protocol a signal of a label attached to a probe can become detectable after said probe has hybridised to a complementary target nucleic acid and when said target has exceeded a threshold level; the time or cycle number to positivity. In other methods for quantification, the time to positivity is used for quantification without addition of a competitor⁷.
Alternatively, the original amount of nucleic acid is established by determining the amplification rate of said nucleic acid during an amplification reaction, as outlined in PCT application PCT/NL03/00780 of the present applicant which is incorporated herein by reference. According to PCT/NL03/00780, a nucleic acid amplification rate is indicative for the amount of nucleic acid initially present in a sample before amplification.

In one embodiment the absolute amount of AC133 expression product is determined. In a preferred embodiment however the relative ratio of an AC133 expression product is determined in relation to another nucleic acid (for instance DNA and/or RNA) or gene product thereof (derivable by transcription and/or translation, such as mRNA and/or a (poly)peptide) present in a sample obtained from said individual. In terms of the invention, by a relative ratio is meant the amount of said AC133 expression product in relation to the amount of said other nucleic acid and/or gene product thereof. Said relative ratio can for instance be determined by (amongst other things) dividing the amount of said AC133 expression product by the amount of said other nucleic acid or gene product thereof, or vice versa. The amount of one or both products can also be divided by, or subtracted from, a reference value. Preferably, said other nucleic acid and/or gene product thereof comprises nuclear nucleic acid and/or gene product thereof. Nuclear nucleic acid, as defined herein, comprises chromosomal DNA and/or RNA transcribed therefrom. More preferably, said other nucleic acid and/or gene product thereof is stable and/or abundantly present in a cell, such as DNA or corresponding mRNA encoding components of small nuclear ribonucleoprotein (snRNP), and/or other essentially common nucleic acid or gene product thereof derived from chromosomal DNA. In one embodiment, said other nucleic acid or gene product thereof comprises U1A or Beta-Actin RNA. When the number of AC133 mRNA copies is compared to the number of nuclear nucleic acid or gene product thereof, the amount of AC133 expression product is preferably expressed as copies per cell.
For instance, if said AC133 expression product comprises protein, a relative ratio between said AC133 protein and at least one other protein in said sample is preferably determined. Said relative ratio is preferably compared with a reference value. Said other protein is preferably abundantly present. For instance, the relative ratio between said AC133 protein and a (preferably abundantly present) house keeping protein is determined. In another embodiment a relative ratio between the amount of AC133 in a sample and the amount of total protein in a sample is determined.
If said AC133 expression product comprises RNA, preferably mRNA, a relative ratio between said AC133 RNA and at least one other nucleic acid in said sample is preferably determined. Said relative ratio is preferably compared with a reference value. Said other nucleic acid is preferably abundantly present. In one embodiment said other nucleic acid comprises U1A. In another embodiment a relative ratio between AC133 RNA, preferably mRNA, in a sample and the amount of total nucleic acid (be it total RNA, total DNA or total RNA + DNA) in a sample is determined.

In one embodiment, said AC133 expression product and said other nucleic acid or gene product thereof both comprise nucleic acid. Minute amounts of target nucleic acid can be detected and quantified by using enzymatic amplification reactions, such as for instance (RT)-PCR, NASBA, SDA, TMA, bDNA or Rolling Circle amplification. In one embodiment, both kinds of nucleic acid are amplified separately and the initial amounts are determined separately. Preferably, however, both nucleic acid sequences are amplified in the same assay (called herein a duplex amplification reaction) because in that case double spreading in the result is avoided, as outlined in WO 02/46470 of the present applicant.
Generally, double spreading in a result is obtained due to varieties in conditions in different reaction mixtures. For instance, with amplification reactions, the temperature of the reaction mixture of nucleic acid 1 may be slightly higher than the temperature of the reaction mixture of nucleic acid 2. This may result in a higher yield of nucleic acid 1 and, hence, in a higher ratio of the amount of nucleic acid 1 versus nucleic acid 2 than would have been obtained if the temperature of reaction mixture 1 had been exactly the same as the temperature of reaction mixture 2. Because of said temperature difference in said reaction mixtures, the determined ratio is not exactly the same as the real ratio of the two nucleic acids present in the initial sample. Likewise, minute variations in other conditions like for instance the amount of enzyme added can lead to variations in the determined amounts of nucleic acids 1 and 2. Thus, in separate amplification reactions, the measured amounts of nucleic acids 1 and 2 may vary independently from each other. Independent variations in said determined amounts may result in variation in a calculated ratio of said measured amounts. This is called the double spreading in the result. Thus, by double spreading is meant herein at least one variation in an obtained result, due to a variety of at least one reaction condition in at least two reaction mixtures. For instance, the temperature and/or the total amount of volume may differ slightly between two reaction mixtures.
Double spreading is for instance prevented by determination of said ratio in the same assay. This means that a processing step and/or a measurement of the amounts of at least 2 nucleic acids and/or gene products thereof is performed in the same assay. In terms of the invention, an assay typically utilises one reaction mixture. Preferably, all components of an assay of the invention are mixed randomly in said assay. Said reaction mixture is preferably present in one reaction tube.
However, a person skilled in the art can think of more methods to prevent double spreading in the result. He/she can for instance use a reaction vessel which is divided in different parts by a (semi)permeable membrane. As long as at least one reaction condition varies dependently in said different parts, double spreading is avoided and the obtained result will be even more accurate.
In one embodiment of the current invention AC133 RNA and a second nucleic acid are amplified in one assay. When both nucleic acid sequences are amplified in one assay, the same varieties in reaction conditions in said assay will influence the obtained amount of each sequence. For instance, the obtained amount of each sequence present in said assay will be influenced by the same temperature, the same overall volume, and so on. Detection of said two target sequences can be achieved by using two specific probes during the generation of amplified nucleic acids in an amplification reaction. Preferable said two probes each have a different label allowing discrimination between said two probes and thereby between said two different target sequences. Quantification is for instance achieved by relating the time to positivity as well as the slope of the relative fluorescence increase of both real time amplification reactions. Preferably, a reference curve is created before. The quantification of said nucleic acid is then performed by comparing the obtained value(s) with said reference curve. Thus there is no need for an internal standard like for instance a competitor molecule. A method of relative quantification of two targets in one assay has an improved accuracy compared to quantification in two separate assays, and requires less handling time and reagents. Duplexing of two amplification reactions in the same tube gives an immediate indication of the ratio of the two targets. In one embodiment, dividing one amount of nucleic acid by another is performed by dividing the intensity of the corresponding fluorescent label by another.

In a preferred embodiment of the invention, at least one sample from said individual is obtained before said treatment and at least one sample from said individual is obtained after initiation of said treatment. In a more preferred embodiment, several samples from said individual are obtained at different time points after initiation of treatment. This enables monitoring the course of treatment during a prolonged period. It can for instance be determined whether the amount of AC133 expression product remains indicative for said disease or the treatment thereof. This is for instance useful for establishing appropriate treatment schedules, dosage and type on a patient per patient basis. Furthermore it can be determined whether continuation of treatment at a given time point is appropriate. For instance, during tumor treatment, the amount of AC133 mRNA drops. If the amount of AC133 mRNA remains low as compared to the amount of AC133 mRNA in a sample obtained before treatment, it indicates that the treatment remains effective. If however the amount of AC133 initially drops, but subsequently raises again, this indicates that the effectiveness of the therapy diminishes. In that case, the dosage of the medicament(s) is optionally increased. If the amount of AC133 mRNA lowers again after an increased dosage, it indicates that such higher dosage is more effective in counteracting disease. If however the amount of AC133 expression product does not lower, and/or does not remain low, one may decide to stop the therapy. Another therapy, if available, is chosen which is also monitored with a method of the invention.
In general, as long as measurements at different time points indicate that an AC133 expression product is altered as compared to the amount of AC133 expression product in a comparable sample taken before therapy, it indicates that the therapy is effective.

With a method of the invention it is possible to determine whether a treatment is effective in an individual. This can be done while a treatment is given or shortly after said treatment or part thereof has ended. Thus it is possible for instance to adjust treatment schedule, dosages and type on a patient per patient basis. It is preferred that said sample is obtained within a month of initiation of treatment. More preferably, said sample is obtained within a week, most preferably within two days of initiation of treatment because an early estimation of effectiveness of therapy allows for early adjustment of treatment schedule, dosages and type. With a method of the invention it is possible to evaluate treatment effectiveness almost immediately after initiation of said treatment, especially when the amount of AC133 mRNA is determined. A method of the invention thus allows easy, early monitoring of treatment, whereas current methods such as analyzing biopsy samples and radiological analysis of tumor cells require complicated, expensive and/or time-consuming procedures.

If the (mean and/or relative) amount of AC133 expression product in a sample of an individual is compared to a reference value (such as for instance the amount of AC133 expression product in a comparable sample of the same individual before the start of therapy, or a mean value of comparable samples of a healthy and/or diseased population), the difference between said reference and said (mean and/or relative) amount of AC133 expression product in said sample is preferably greater than or equal to the standard deviation of the reference. More preferably, said difference is greater than or equal to two times the standard deviation of the reference. Most preferably, said difference is greater than or equal to three times the standard deviation of the reference. If a (PBMC) sample is used without further significant purification of cells, the amount of AC133 expression product in said sample is preferably at least two times higher or lower (depending on whether disease or treatment of disease is measured) than said reference value. More preferably, the amount of AC133 expression product in said sample is at least four times higher or lower than said reference value. Most preferably, the amount of AC133 expression product in said sample is at least ten times higher, or lower, than said reference value. Of course, the (absolute) difference between the amount of AC133 expression product in a sample of an individual and a reference value is dependent on the specific kind of sample used and/or on the relative ratio of the amounts of AC133 expression product and another expression product.

The difference in the (relative) amount of AC133 expression product in an effective and a non-effective treatment can be very large. In the extreme cases the level of AC133 expression product ranges from detectable to not detectable. A zero to one relation can be used to design relatively simple test systems. A zero to one relation is of course dependent on the detection system used to detect AC133 expression product. Very sensitive expression detection systems will typically detect expression product where a less sensitive systems detects no expression product. A person skilled in the art is well capable of designing the most appropriate expression detection system to practice this preferred embodiment of the invention.

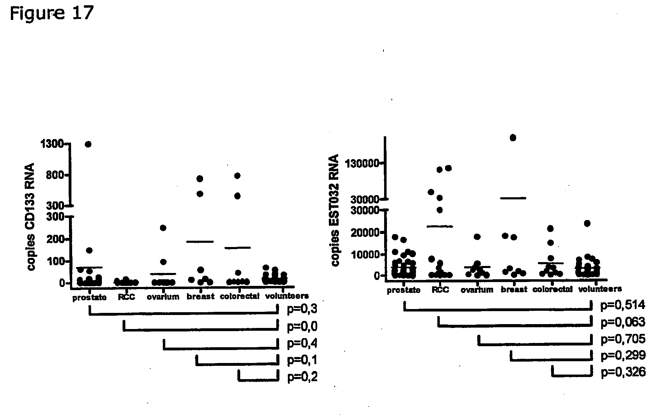

In one embodiment a method of the invention is provided wherein said disease comprises the presence of a tumor. Accordingly the amount of AC133 expression product is altered in an individual suffering from, or at risk of suffering from, a tumor related disease as compared to the amount of AC133 expression product in an individual who is not, or to a significantly lesser extent, suffering from, or at risk of suffering from, a tumor related disease. Furthermore, a method of the invention is suitable for determining whether a tumor is progressive. A progressive tumor is defined herein as a tumor with a significantly growing tumor mass and/or a tumor which is involved in the development and/or presence of at least one metastasis and/or circulating tumor cells originating from said tumor. Contrary, the mass of a stable tumor is slowly, if at all, growing and a stable tumor is not or barely involved in development of metastases. According to the present invention, a (relative) amount of AC133 expression product is higher when a tumor is progressive as compared to a stable tumor. In one embodiment a method of the invention is therefore used for determining whether an individual is suffering from, or at risk of suffering from, a progressive tumor. This embodiment is particularly suitable if an individual is suffering from a type of tumor which is generally involved in a modest increase of AC133 expression product, as compared to other kinds of tumors. According to the invention, various kinds of tumors are involved with different increases in AC133 expression product. This is for instance shown in Figure 17. Breast cancer and colorectal cancer are involved in higher increases in AC133 expression product as compared to ovarium cancer, prostate cancer and renal cell carcinoma. Hence, especially when an individual appears to be suffering from a type of tumor which is involved in a relatively modest increase of AC133 expression product, a high amount of AC133 expression product indicates that said tumor is progressive.

In another preferred embodiment a method is used for monitoring the status of a tumor over time. In this embodiment the amount of AC133 expression product is determined in at least two samples taken from an individual at different time points. If the amount of AC133 expression product in an individual appears to be declining over time, it indicates that said tumor has become (more) stable, and/or that regression has occurred. A declining amount of AC133 in an individual is for instance determined when the amount of AC133 expression product in a sample taken from said individual at a later time point comprises less AC133 expression product as compared to the same kind of sample taken from said individual at an earlier time point. If however the amount of AC133 expression product in an individual appears to be (suddenly) raising, it indicates that a tumor has become more progressive, and/or that a cured patient experiences a cancer relapse. In one embodiment a method of the invention is therefore used for monitoring the status of a tumor. In a preferred embodiment a method of the invention is used for monitoring the status of a tumor during treatment, in order to assess whether a treatment is effective in at least partly counteracting said tumor. If a tumor becomes less progressive, a treatment is effective. Moreover, a method of the invention is preferably used after tumor treatment, for instance in order to monitor whether a stable tumor remains stable or becomes progressive, and/or to monitor whether a (progressive) tumor evolves (for instance whether a cured individual experiences a cancer relapse). The invention thus provides a method for determining whether a treatment of an individual is effective in at least in part counteracting a progressive tumor, comprising determining whether a sample from said individual comprises a lower (relative) amount of AC133 expression product as compared to the same kind of sample of said individual taken at an earlier time point.
In a preferred embodiment the (relative) amount of AC133 RNA, preferably mRNA, in a sample is determined. This is preferably performed using a nucleic acid molecule comprising at least part of a sequence of AC133 or an analogue thereof as a primer and/or probe. The length of said nucleic acid molecule or analogue is preferably at least 4 nucleotides.

Preferably, said length is at least 5 nucleotides, more preferably at least 6 nucleotides, more preferably at least 7 nucleotides, more preferably at least 8 nucleotides, more preferably at least 9 nucleotides, most preferably at least 10 nucleotides in order to enhance specificity of said primer and/or probe. Said nucleic acid molecule or analogue is furthermore preferably shorter than 150 nucleotides in order to allow efficient binding. Said nucleic acid molecule or analogue preferably comprises between about 15 and about 30 nucleotides. The length of said part of a AC133 sequence is preferably at least 50%, more preferably at least 60%, more preferably at least 70% more preferably at least 80%, more preferably at least 90%, most preferably at least 95% of the length of said nucleic acid molecule. One embodiment thus provides a use of a nucleic acid molecule comprising at least part of a AC133 sequence or an analogue thereof for determining whether a tumor is progressive.
A use of a nucleic acid molecule comprising at least part of a sequence of AC133 or an analogue thereof for monitoring a treatment of an individual suffering from a disease comprising the presence of a progressive tumor is also herewith provided.
Alternatively, or additionally, it is possible to use another kind of molecule capable of specifically binding an AC133 expression product. The description therefore also provides a use of a molecule capable of specifically binding an AC133 expression product for determining whether a tumor is progressive.

Moreover, treatment of said tumor-related disease can be monitored with a method of the invention. In a preferred embodiment, said tumor comprises mouth bottom carcinoma, adenoidcystic carcinoma, renal cell carcinoma, colon carcinoma, an esophagus tumor, mesothelioma, pancreas tumor, bladder tumor, adenocarcinoma of unknown primary (ACUP), prostate tumor, renal adenocarcinoma, head and neck cancer and/or malignant melanoma. As is shown in the examples, the amount of AC133 mRNA is significantly lowered during treatment of these diseases. It is also shown in the examples that the expression of AC133 per 10,000 copies U1A DNA or Beta-Actin DNA in various untreated cancer patients is almost one log higher compared to healthy donors. Accordingly, the extent of increase in AC133 expression product is dependent on the kind of tumor.
Therefore, in a further preferred embodiment said tumor comprises breast cancer, colorectal cancer, prostate cancer and/or ovarium cancer because these types of tumor are particularly associated with an increase of AC133 expression product, as is shown in figure 17. Most preferably, said tumor comprises breast cancer and/or colorectal cancer, since these tumors are involved in high increases of the amount of AC133 expression product.

In yet another embodiment, a method is provided wherein said disease is a blood vessel related disease. As used herein, by a blood vessel related disease is meant a disease which involves generation, maintenance and/or breakdown of blood vessels. Preferably, said disease comprises heart disease, high blood pressure, transient ischemic attacks and strokes, psoriasis, Crohn's disease, rheumatoid arthritis, endometriosis, atherosclerosis, obesity, diabetes, diabetic rethinopathy, macular degeneration, Alzheimer disease, Peutz-Jegher's syndrome, multiple sclerosis, systemic lupus erythematosus, Wegener's granulomatosis, vasculitis, sickle cell disease, thallassemia and/or angina. A healing process can be followed with a method. For instance, recovery of damaged tissue involves an altered amount of AC133 expression product over time. Samples taken at different time points provide information about the amount of AC133 expression product that is generated during different time intervals. An altered amount of AC133 expression product found in samples during a period of time is for instance indicative for generation of tissue cells. An important application is treatment of heart and coronary disease. A method is suitable for monitoring the generation of new cardiac tissue.

In one aspect a method of the invention is provided wherein said sample emprises a significant amount of non-endothelial cells. It has been shown by the present inventors that the number of circulating endothelial cells is hot always indicative for the status of an individual, while the total amount of AC133 expression product is indicative for said status. Preferably, said sample is an essentially cell-free sample.
In a preferred embodiment, a sample of a method of the invention is a blood sample, although the location of for instance an angiogenic process can be a tumor or a part of the skin. A blood sample is preferred, amongst other things because it is much easier to obtain and relative large amounts are often available. A blood sample is also often easier to investigate, requiring less expensive and/or specific equipment. Quite surprisingly, we have found that the expression of AC133 by hematopoietic cells, like peripheral blood mononuclear cells (PBMCs), is indicative for a process occurring somewhere else in an individual's body. For instance, the alteration in amount of an AC133 expression product in PBMCs is indicative for the presence of a tumor somewhere in the body, or for the treatment thereof. The amount of an AC133 expression product in PBMCs provides adequate information about different aspects and/or processes of an individual's body. Therefore, in a preferred embodiment, a method of the invention is provided wherein said sample comprises a peripheral blood mononuclear cell.

With a method of the description it is possible to determine whether an individual suffers from or is at risk of suffering from a disease. Moreover it is possible to monitor therapy. Preferably, treatment of a tumor related disease and/or a blood vessel related disease is monitored with a method of the invention.
If a disease involves the presence of a tumor and/or an elevated level of angiogenesis, treatment typically comprises counteracting said tumor and/or said angiogenic process. Since such treatment is now easily monitored by a method of the invention, it is likewise easy to determine whether said treatment is effective. In the art, many drugs are known for anti-tumor and/or anti-angiogenic treatment such as the following drugs: 2ME2, ABT510, ABT751, Angiostatin, Angiozyme, Anti-VEGF RhuMAb, Apra (CT-2584), Avicine, Benefin, BMS275291, Carboxyamidotriazole, CC4047, CC5013, CC7085, CDC801, CGP-41251 (PKC 412), CM101, Combretastatin A-4 Prodrug, DMXAA, EMD 121974, Endostatin, Enzastaurin HCl, Flavopiridol, Genistein (GCP), Green Tea Extract, IM-862, ImmTher, Interferon alpha, Interleukin-12, Iressa (ZD1839), LY317615, Marimastat, Metastat (Col-3), Neovastat, Octreotide, Paclitaxel, Penicillamine, Photofrin, Photopoint, PI-88, Prinomastat (AG-3340), PTK787 (ZK22584), RO317453, Solimastat, Squalamine, SU 101, SU11248, SU 5416, SU-6668, Suradista (FCE 26644), Suramin (Metaret), Tetrathiomolybdate, Thalidomide, TNP-470, VEGF trap, ZD6126 and/or Vitaxin. Thus, in one embodiment of the invention a method of the invention is provided wherein said treatment comprises the use of at least one of these drugs. However, the artisan can think of more drugs that can be used during said treatment.

Now that there is a method for diagnosis of disease and/or monitoring of treatment of a disease, candidate compounds or methods can be tested for beneficial activity and/or possible side effects. Additionally, it can be tested whether compounds or methods are involved with causing/enhancing disease. Similar methods of the invention are performed for this purpose: after administration of such candidate compound to an individual, the amount of AC133 expression product in a sample from said individual is determined. If said amount of AC133 expression product is less indicative for disease as compared to the amount of AC133 expression product present in a sample of said individual prior to administration of said candidate compound, it indicates beneficial activity of said candidate compound. If, however, said amount of AC133 expression product is more indicative for disease as compared to the amount of AC133 expression product present in a sample of said individual prior to administration of said candidate compound, possible side effects (which may comprise involvement in causing/enhancing disease) of said candidate compound is indicated. The description thus provides a method for determining therapeutic activity and/or possible side-effects of a candidate compound comprising determining whether a sample from an individual, said individual having been provided with said candidate compound, comprises an expression product of AC133 in an amount that is indicative for disease or treatment thereof. A method is also suitable for (selective) toxin testing. The toxic activity of a candidate compound can be determined with a method of the invention. This way, the usefulness of such candidate compound for causing malfunctioning of a cellular organism, for instance by having a cytostatic or cytotoxic effect can be determined.
In one aspect, therapeutic activity, possible side effects and/or toxic activity of a candidate compound is determined by administering said candidate compound to an essentially related organism, such as belonging to the same species or genus, and determining the amount of AC133 expression product in a sample from said essentially related organism. If said amount of AC133 expression product is indicative for disease or the treatment thereof, this also indicates toxic activity, side-effects or therapeutic activity involved with said candidate compound in an essentially related organism. Therefore, for determining therapeutic activity, side-effects and/or toxic activity of a candidate compound, it is not necessary to use exactly the same kind of organism in a method of the invention. An essentially related organism can also be used.

In one aspect is provided a method for typing a sample of an individual suffering from, or at risk of suffering from, a disease comprising obtaining a sample from said individual and determining whether said sample comprises an expression product of AC133 in an amount that is indicative for said disease or for the treatment thereof.

The invention also provides a method for monitoring treatment of an individual suffering from a disease comprising obtaining a sample from said individual and determining whether said sample comprises an expression product of AC133 in an amount that is indicative for said treatment.

In a preferred embodiment a method of the invention is performed with at least one primer and/or probe as depicted in table 2, or a functional part or derivative thereof. If at least one of said primers and/or probes is used, the sensitivity and reliability of a method of the invention is further improved.

In yet another aspect there is provided a use of a nucleic acid molecule comprising at least part of a sequence of AC133, or of an analogue of said nucleic acid molecule, for monitoring a treatment of an individual suffering from a disease. The presence of AC133 RNA in a sample of an individual can be detected by determining whether an AC133 nucleic acid or analogue thereof is capable of specifically hybridizing with RNA in a sample of said individual, preferably after amplification of said RNA of said sample. As defined herein, an AC133 nucleic acid or analogue thereof means a nucleic acid molecule comprising at least part of a sequence of AC133, or an analogue of said nucleic acid molecule. If hybridisation takes place, it is indicative for the presence of AC133 RNA in said individual. The (relative) amount of AC133 RNA can be determined using known methods in the art, as described above. Hence, a nucleic acid molecule comprising at least part of a sequence of AC133 can be used in a method of the invention involving determining the amount of AC133 RNA in a sample. If one sample is obtained after the start of treatment or, preferably, if several samples are obtained after the start of treatment at different time points, said nucleic acid molecule comprising at least part of a sequence of AC133 or said analogue thereof is used for monitoring said treatment. Of course, as is known by a person skilled in the art, a coding strand of DNA/RNA is capable of hybridizing with the complementary strand of a corresponding doublestranded nucleic acid sequence. Hence, a complementary strand of a certain coding strand is particularly suitable for detection of expression of said coding strand.

A part of a nucleic acid sequence of AC133 is defined herein as an AC133 nucleic acid sequence comprising at least 20 nucleotides, preferably at least 30 nucleotides, more preferably at least 50 nucleotides. A part and/or an analogue of an AC133 expression product is defined herein as a part and/or analogue that can be detected using essentially the same kind of detection method capable of detecting said expression product, although the sensibility of detection may differ. An analogue of an AC133 RNA or DNA molecule is defined herein as an RNA or DNA sequence which is at least 50 % homologous to an AC133 RNA or DNA molecule. Preferably, said analogue is at least 60 %, more preferably at least 70 %, more preferably at least 75 %, more preferably at least 80 %, more preferably at least 85 %, more preferably at least 90%, more preferably at least 95 %, most preferably at least 98% homologous to an AC133 RNA or DNA molecule, comprising at least part of a sequence of AC133. In one embodiment said analogue of an AC133 RNA or DNA molecule has essentially the same properties as an AC133 RNA or DNA molecule in kind, albeit not necessarily in amount. A nucleotide mutation, replacement, alteration, addition and/or deletion may have taken place naturally and/or may have been introduced artificially, without essentially altering the detection capability of said analogue as compared to the detection of said AC133 RNA or DNA sequence. A person skilled in the art is well able to determine whether a given RNA or DNA sequence is an analogue of an AC133 RNA or DNA sequence, using techniques known in the art.

Also provided is a diagnostic kit comprising at least one means for performing a method according to the invention, said kit comprising a nucleic acid molecule comprising at least part of a sequence of AC133, said part comprising at least 20 nucleotides. In one embodiment said part comprises at least 30 nucleotides. In another embodiment said part comprises at least 50 nucleotides. Preferably, said kit further comprises suitable means for performing a nucleic acid amplification reaction. Amplification of AC133 mRNA is preferred because amplification of AC133 mRNA enables detection of small initial amounts of AC133 mRNA in a sample. Moreover, an amount of mRNA more accurately reflects the actual status of AC133 expression because of its short half-life. In a preferred embodiment said nucleic acid amplification reaction comprises NASBA, PCR, RT-PCR, TMA, bDNA, SDA or Rolling Circle amplification. In a more preferred embodiment, said nucleic acid amplification reaction comprises NASBA. Suitable primers and probes for amplifying and/or detecting AC133 RNA are listed in table 2. These primers and probes are capable of amplifying and/or detecting both AC133-1 and AC133-2 isoforms. In one aspect the invention therefore provides a diagnostic kit of the invention comprising at least one primer and/or probe as depicted in table 2 or an analogue of said primer and/or probe.

A diagnostic kit is particularly useful for carrying out a method of the invention. In yet another aspect the invention therefore provides a use of a diagnostic kit of the invention for typing a sample of an individual suffering from, or at risk of suffering from, a disease. A use of a diagnostic kit of the invention for monitoring treatment of an individual suffering from a disease is also herewith provided.
Furthermore is provided a primer and/or probe comprising a nucleic acid sequence as depicted in table 2, or a functional part or analogue thereof. Said primer and/or probe is particularly useful for performing a method of the present invention.

The present invention is further explained in more detail by the following examples, which do not limit the invention in any way.

### Examples

### Example 1

### Patients and samples Angiostatin study

5 cancer patients (characteristics depicted in table 1) who were not cured by treatment with other drugs were included in a phase I clinical trial of recombinant human angiostatin (rhAngiostatin). In this trial, designed to determine the toxicity of the drug, patients were treated with 7.5 mg/m²/day rhAngiostatin subcutaneously in a twice-daily schedule. Blood samples of the patients were taken at day 1 and day 28.

### Example 2

Peripheral blood mononuclear cells (PBMC) were isolated and approximately 1x10⁶ cells were dissolved in 1 ml L6 and stored at -80°C. 300 µl of the lysed-PBMC solution (containing approximately 300,000 PBMC) were added to a 1.5 ml eppendorf tube containing 700 µl lysis buffer. The nucleic acid now present in the lysis buffer was further purified with the method described by Boom et al¹. The isolated nucleic acid was eluted in 50 µl elution buffer. Usually a dilution was made such that the equivalent of 10,000 cells/5 µl was used as input in NASBA amplification reactions.

In table 2 the primers and probes used in these examples are summarized. Standard NASBA nucleic acid amplification reactions were performed in a 20 µl reaction volume and contained: 40mM Tris-pH 8.5, 90mM KCl, 12mM MgCl₂, 5mM dithiotreitol, 1mM dNTP's (each), 2mM rNTP's (each), 0.2µM primer P1, 0.2µM primer P2, 0.05µM molecular beacon, 375mM sorbitol, 0.105 µg/µl bovine serum albumin, 6.4 units AMV RT, 32 units T7 RNA polymerase, 0.08 units RNase H and input nucleic acid. The complete mixture, except the enzymes was, prior to adding the enzymes, heated to 65°C in order to denature any secondary structure in the RNA and to allow the primers to anneal. (In the case of Beta-Actin, 2 units of MSP II were added. The mix was incubated at 37°C for 15 minutes, followed by denaturation at 95°C.) After cooling the mixture to 41°C the enzymes were added. The amplification took place at 41°C for 90 min in a thermostated fluorimeter (CytoFluor 2000 or EasyQ Reader) and the fluorescent signal of the molecular beacon probe was measured every 45 seconds.
To achieve quantification, a dilution series of target sequence was amplified and the time points at which the reactions became positive (the time to positivity, TTP) were plotted against the input amounts of nucleic acid. This way a calibration curve was created that could be used to read TTP values of reactions with unknown amounts of input and deduce the input amount.

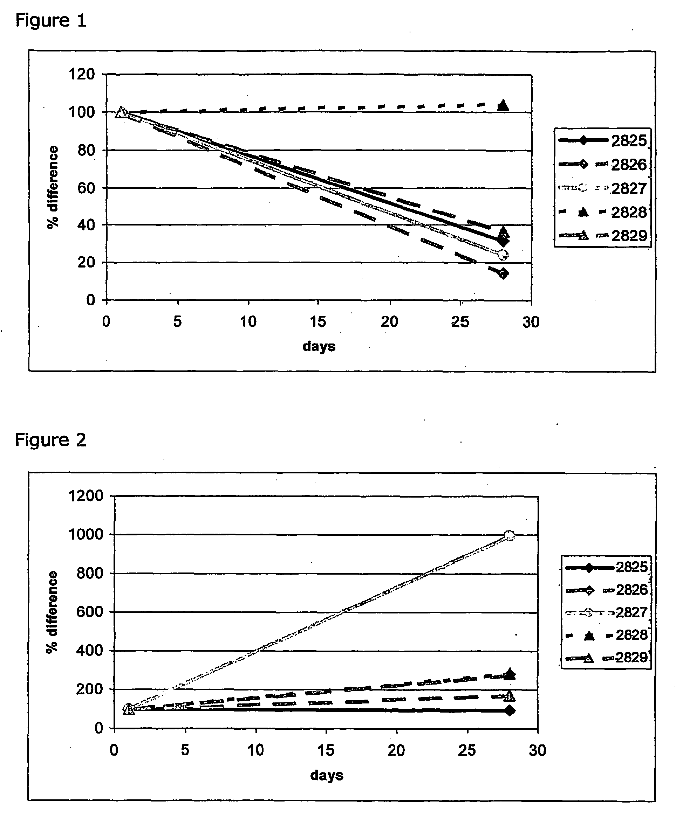

The AC133 expression per 10,000 cells was calculated after determination of the AC133 expression and the Beta-Actin copy number for each sample. The results are shown in figure 1. From this figure it is clear that the AC133 expression per 10,000 cells in 4 of the 5 patients drops significantly. There seems to be no correlation with the kind of tumor, rate of progression (Table 1) or the total number of circulating endothelial cells (CECs; Figure 2, quantified according to Beerepoot, L.. Ann.Oncol., 15: 139-145, 2004).

### Example 3

### Patients and samples PrimMed01 study

For this study samples of 14 patients were available, but since we had no pre-treatment sample of two patients, they were not included in the analysis. The characteristics of the remaining 12 patients are depicted in table 3. Patients received daily treatment with PrimMed01 (protein kinase inhibitor; antiVEGF) during 8 days. After this pre-treatment, the daily treatment with PrimMed01 was continued, but additionally patients received a course of gemcitabine and cisplatin on day 15 (course 1) and day 36 (course 2).
Blood samples were taken before and after pre-treatment, before each course, and after 0, 2, 4, 8, and 24, hours after each course. After the first course an extra sample was taken after 48 hours.

### Example 4

Isolation of nucleic acids from PBMC and NASBA amplification were performed as described in example 2 with one modification: U1A was used instead of Beta Actin.

For the patients in the PrimMed01 study not only the expression of AC133 per 10,000 cells, but also the expression of EST032 per 10,000 cells was determined.

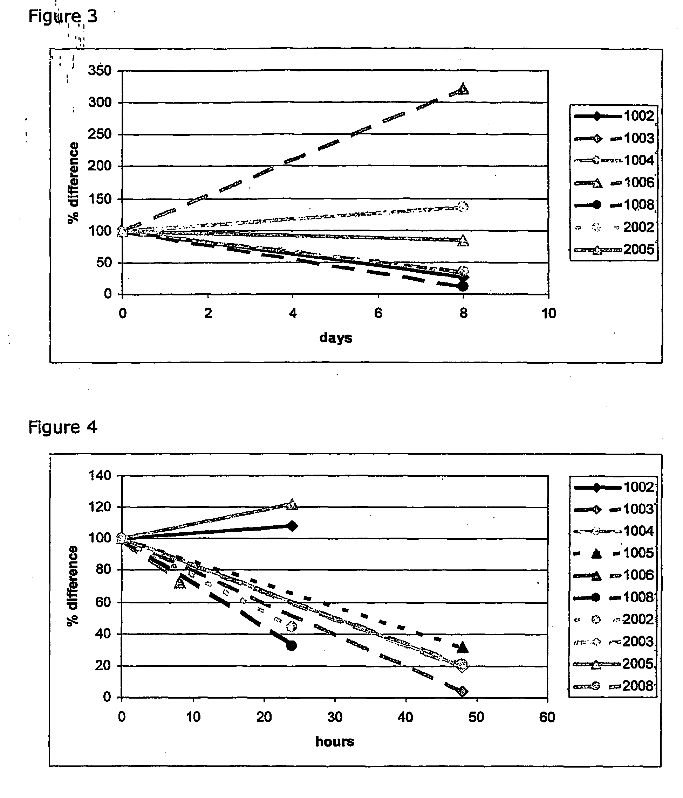

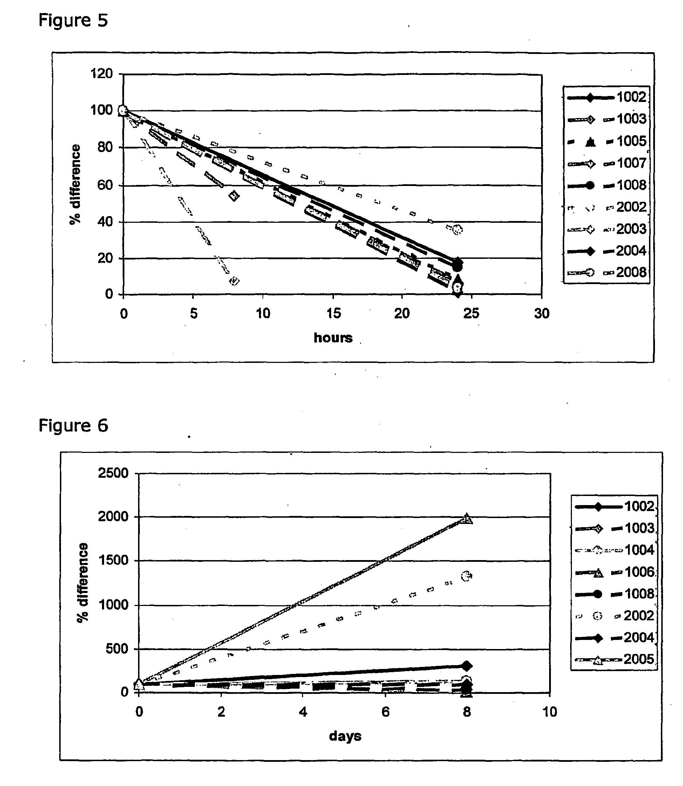

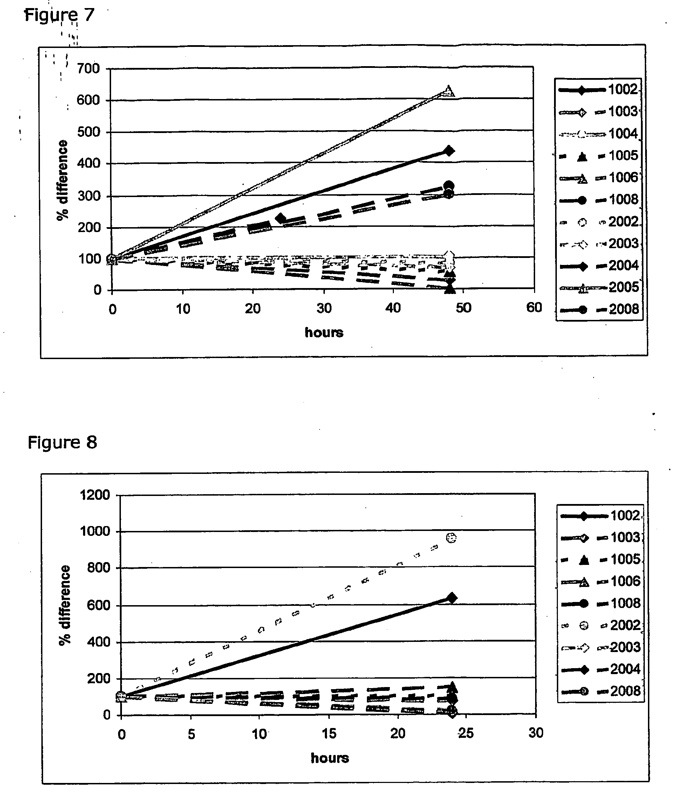

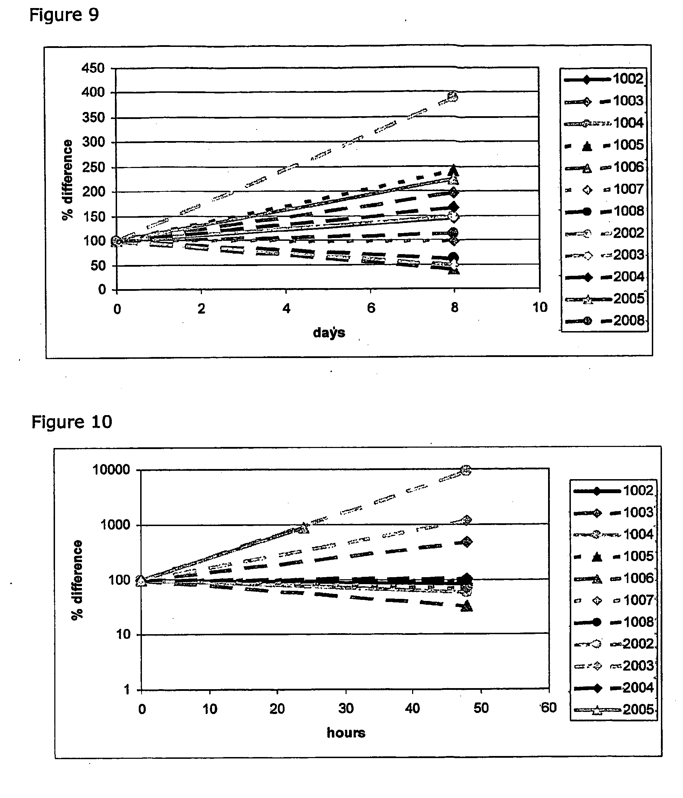

From the figures 3-5 it is clear that AC133 expression drops significantly during treatment with PrimMed01 and gemcitabine and cisplatin. During the pre-treatment the AC133 expression does not seem to be affected, but here the observation period is much longer (8 days) compared to course 1 (follow up 48 hours) and course 2 (follow up 24 hours).
If we look at figures 6-8 we clearly see that the expression of EST032 is not affected by therapy. It is therefore concluded that the decrease of AC133 expression is a specific effect of the therapy on the expression of AC133, and is not caused by the method.
As is shown in figures 9-11, there is also no association between AC133 expression and the amount of CECs.

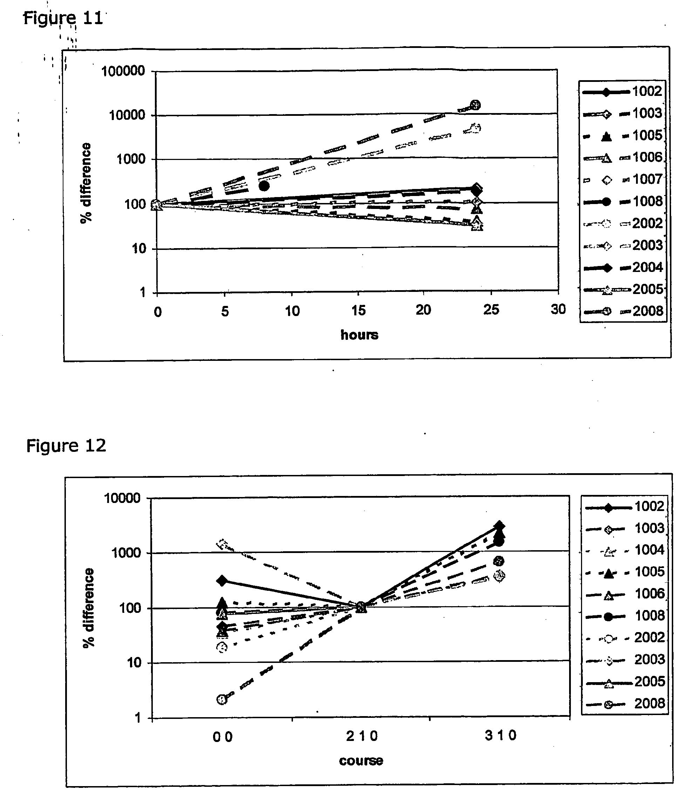

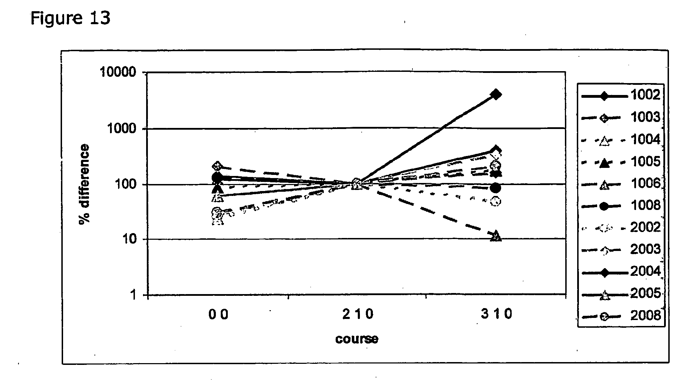

In figures 12 and 13 we look at the effect of therapy over a longer period of time. In figure 12 the relative expression of AC133 just before each course is plotted (day 1, 15, and 35). After effective therapy (as determined in figure 4) an increase in AC133 expression is shown. That this effect on AC133 expression is a specific effect, and not caused due to the methods used, becomes clear if we look at the EST032 expression in the same samples (Figure 13).

### Example 5

### Patients and samples healthy donors versus untreated patients

For this study samples of 54 individuals were available, of which 8 samples were from healthy donors and 46 were from patients who were not receiving any anticancer treatment at the moment of blood sampling.

### Example 6

Isolation of nucleic acids from PBMC and NASBA amplification were performed as described in example 4.

In table 4 we see that in cancer patients the expression of AC133 per 10,000 cells is almost one log higher compared to healthy donors. The difference in expression of EST032 between these two groups is much smaller.

### Example 7

The experiment of Example 6 was repeated with a higher amount of samples. For this study samples of 174 individuals were available, of which 29 samples were from healthy donors and 145 were from patients who were not receiving any anticancer treatment at the moment of blood sampling.

Isolation of nucleic acids from PBMC and NASBA amplification were performed as described in example 4.

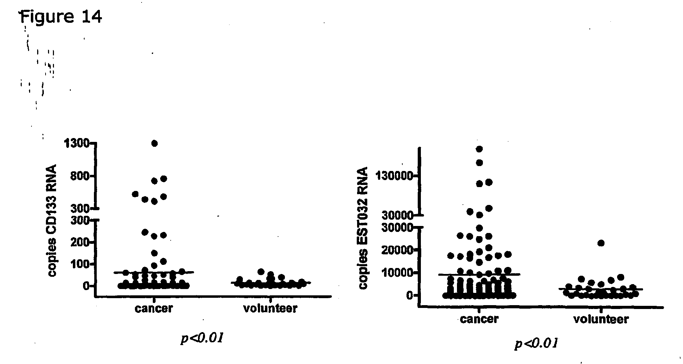

In Figure 14 we see that in cancer patients the expression of AC133 per 10,000 cells is higher compared to healthy donors. The same holds true for difference in expression of EST032.

### Example 8

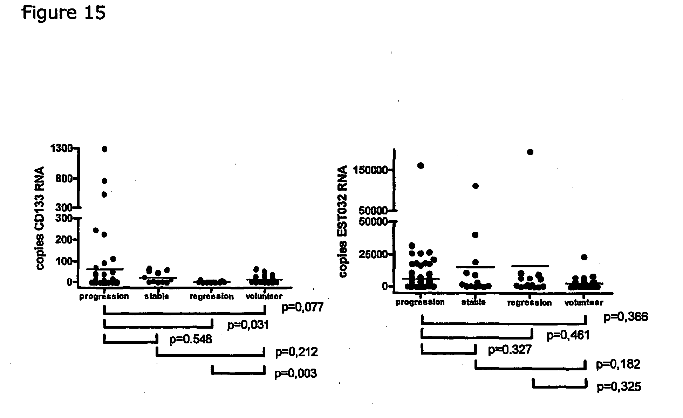

The patients from the previous examples differ for example in the way their cancer progresses. If all samples are divided in groups according to cancer growth (progressive disease, stable disease, regression, and healthy volunteer; Figure 15),no differences in EST032 expression are found between the groups. However if AC133 mRNA expression is compared, significant differences are found between patients with progression or regression, and volunteers and patients with regression.

### Example 9

Another way in which the samples from the previous examples can be analysed, is by dividing patients according to the type of their disease. As shown in Figure 17, especially patients with renal cell carcinoma (RCC) have a significantly increased expression of EST032 and AC133.

### Example 10

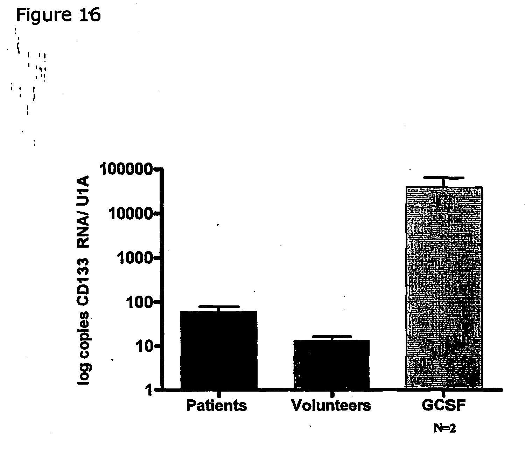

Two samples of patients who have been subjected to treatment with GCSF were tested. GCSF mobilizes stem cells from the bone marrow, so it is expected to find more AC133 expressing cells in the blood after a treatment with GCSF. It is clear from Figure 16 that the AC133 expression in these 2 patients is much higher compared to volunteers and all the other patients.

### Tables

**Table 1. Characteristics of patients Angiostatin study**

| **patient number** | **age** | **tumor type** | **progression rate** |
|---|---|---|---|
| 2825 | 41 | mouth bottom carcinoma | progressive |
| 2826 | 49 | adenoidcystic carcinoma | stable |
| 2827 | 49 | adenoidcystic carcinoma | stable |
| 2828 | 72 | renal cell carcinoma | stable |
| 2829 | 54 | colon carcinoma, liver/lung metastases | progressive |

**Table 2. Sequences of primers and probes used.**

| **Name** | **Sequence¹** |
|---|---|
| U1A P1 | 5' *AAT TCT AAT ACG ACT CAC TAT AGG* GAG AGG CCC GGC ATG TGG TGC **ATA A 3'** |
| U1A P2 | 5' CGC GCC TCT TTC TGG GTG T**T 3'** |
| U1A MB | 5' CGC ATG CTG TAA CCA CGC ACT CTC CTC **GCA TGC G 3'** |
| AC133 P1 | 5' *AAT TCT AAT ACG ACT CAC TAT AGG* GAA GAA CAG GGA TGA TGT TGG GTC TCA **3'** |
| AC133 P2 | 5' TTT CAA GGA CTT GCG AAC TCT **CTT GA 3'** |
| AC133 MB | 5' CGA TCC AAG GAC AAG GCG TTC ACA **GGA TCG 3'** |
| EST032 P1 | 5' *AAT TCT AAT ACG ACT CAC TAT AGG* GAG TAG CCC ACT CAA GAG CTC TCT CCT GTT GGT CCC T **3'** |
| EST032 P2 | 5' GCA TCT CTG TTC ATG ACT GTG TGA GCT CCT GTC CT 3' |
| EST032 MB | 5' CGC ACG AAT GAC GTG CCC CTG CGA A**TC GTA CG 3'** |

| | |
|---|---|
| 1. The T7 promoter part of primer P1 sequences is shown in *italics*, the stem sequences of the molecular beacon probes (MB) are shown in **bold.** The molecular beacon probes were labeled at the 3' end with DABCYL (the quencher) and at the 5' end with a fluorescent label, which in the case of U1A is ROX, and in the case of AC133 and EST032 is 6-FAM. | |

**Table 3. Characteristics of patients PrimMed01 study**

| **patient number** | **gender** | **PrimMed01 dose (mg)** | **gemcitabine dose (mg/m2)** | **cisplatin dose (mg/m2)** | **tumor type** | **best response** |
|---|---|---|---|---|---|---|
| 1002 | M | 350 | 1000 | 60 | esophagus | stable disease |
| 1003 | M | 350 | 1000 | 60 | mesothelioma | stable disease |
| 1004 | F | 350 | 1000 | 60 | pancreas | - |
| 1005 | F | 350 | 1250 | 60 | pancreas | stable disease |
| 1006 | M | 350 | 1250 | 60 | bladder | stable disease |
| 1007 | M | 350 | 1250 | 75 | pancreas | stable disease |
| 1008 | M | 500 | 1250 | 75 | ACUP | stable disease |
| 2002 | M | 350 | 1250 | 60 | prostate | partial regression |
| 2003 | M | 350 | 1250 | 75 | adeno renal | progressive disease |
| 2004 | M | 350 | 1250 | 75 | head and neck | partial response |
| 2005 | M | 350 | 1250 | 75 | melanoma | progressive disease |
| 2008 | M | 500 | 1250 | 75 | melanoma | partial response |

**Table 4. RNA expression (expressed in log value) in healthy donors vs. untreated patients**

| | | **donors** | **untreated patients** |
|---|---|---|---|
| | number of samples | 8 | 46 |
| **AC133** | average (per 10⁴ U1A) | 2.02 | 2.94 |
| **EST032** | average (per 10⁴ U1A) | 3.29 | 3.97 |

### Brief description of the drawings

**Figure 1****.** Amount of AC133 expression per 10,000 cells (quantified by Beta-Actin) in 5 patients treated with rhAngiostatin. The sample at day 1 (before therapy) was set at 100%.
**Figure 2****.** Number of circulating endothelial cells. The total amount of circulating endothelial cells was quantified by immunomagnetic separation with endothelium specific antibody directed to CD146. The sample at day 1 (before therapy) was set at 100%.
**Figure 3-5****.** Amount of AC133 expression per 10,000 cells (quantified by U1A) in patients treated with PrimMed01 and gemcitabine and cisplatin. The first sample (before therapy) was set at 100%. Only the sample before each course and the latest available sample during that course were plotted. Figure 3: pre-treatment, Figure 4: first course, Figure 5: second course.
**Figure 6-8****.** Amount of EST032 expression per 10,000 cells (quantified by U1A) in patients treated with PrimMed01 and gemcitabine and cisplatin. The first sample (before therapy) was set at 100%. Only the sample before each course and the latest available sample during that course were plotted. Figure 6: pre-treatment, Figure 7: first course, Figure 8: second course.
**Figure 9-11****.** Amount of circulating endothelial cells (CEC) per ml. of blood in patients treated with PrimMed01 and gemcitabine and cisplatin as determined by FACS analysis with CD34. The first sample (before therapy) was set at 100%. Only the sample before each course and the latest available sample during that course were plotted. Figure 9: pre-treatment, Figure 10: first course, Figure 11: second course.
**Figure 12****,** **13****.** Amount of mRNA expression per 10,000 cells (quantified (by U1A) in patients treated with PrimMed01 and gemcitabine and cisplatin. The 3 samples represent the sample before pre-treatment, the sample just before course 1, and the sample just before course 2. The second sample (taken just before course 1) was set at 100%. Figure 12: AC133 expression, Figure 13: EST032 expression.
**Figure 14****.** AC133 and EST032 expression per 10,000 cells (quantified by U1A) in progressive cancer patients and healthy donors. Left panel: AC133, right panel: EST032. The average expression of AC133 and EST032 is significantly increased in patients as compared to healthy donors.
**Figure 15****.** AC133 and EST032 expression per 10,000 cells (quantified by U1A) in all samples, stratified on cancer growth. Left panel: AC133, right panel: EST032. A difference is considered to be significant if p value >0.05.
**Figure 16****.** Average AC133 expression per 10,000 cells (quantified by U1A) in all samples in two patients that were treated with GCSF.
**Figure 17****.** AC133 and EST032 expression per 10,000 cells (quantified by U1A) in all samples, stratified on cancer type. Left panel: AC133, right panel: EST032. A difference is considered to be significant if p value >0.05.

### References

1. Boom R, Sol CJ, Salimans MM, Jansen CL, Wertheim-van DillenPM, Van der Noordaa J: Rapid and simple method for purification of nucleic acids. J.Clin.Microbiol. 28: 495-503, 1990
2. Van Gemen B, van Beuningen R, Nabbe A, Van Strijp D, Jurriaans S, Lens P, Kievits T: A one-tube quantitative HIV-1 RNA NASBA nucleic acid amplification assay using electrochemiluminescent (ECL) labelled probes. J.Virol.Methods 49: 157-167, 1994
3. Heid CA, Stevens J, Livak KJ, Williams PM: Real time quantitative PCR. Genome Res. 6: 986-994, 1996
4. Tyagi S, Kramer FR: Molecular beacons: probes that fluoresce upon hybridization. Nat.Biotechnol. 14: 303-308, 1996
5. Leone G, van Schijndel H, Van Gemen B, Kramer FR, Schoen CD: Molecular beacon probes combined with amplification by NASBA enable homogeneous, real-time detection of RNA. Nucleic Acids Res. 26: 2150-2155, 1998
6. Piatak M, Luk KC, Williams B, Lifson JD: Quantitative competitive polymerase chain reaction for accurate quantitation of HIV DNA and RNA species. Biotechniques 14: 70-81, 1993
7. De Baar, MP, van Dooren, MW, de Rooij, E, Bakker, M, Van Gemen, B, Goudsmit, J, and de Ronde, A. Single rapid real-time monitored isothermal RNA amplification assay for quantification of HIV-1 isolates from group M, N, and O. J. Clin. Microbiol. 39(4): 1378-1384,2001
8. Folkman, J. Angiogenesis in cancer, vascular, rheumatoid and other disease. Nat.Med., 1: 27-31, 1995.
9. Beerepoot, L. V., Mehra, N., Vermaat, J. S., Zonnenberg, B. A., Gebbink, M. F., and Voest, E. E. Increased levels of viable circulating endothelial cells are an indicator of progressive disease in cancer patients. Ann.Oncol., 15: 139-145, 2004.
10. Miraglia S., Godfrey W., Yin A.H., Atkins K., Warnke R., Holden J.T., Bray R.A., Waller E.K., Buck D.W. A novel five-transmembrane hematopoietic stem cell antigen: isolation, characterization, and molecular cloning. Blood. 1997, 90(12):5013-5021.
11. Yin A.H., Miraglia S., Zanjani E.D., Almeida-Porada G., Ogawa M., Leary A.G., Olweus J., Kearney J., Buck D.W. AC133, a novel marker for human hematopoietic stem and progenitor cells. Blood. 1997, 90(12):5002-5012.
12. Yu Y., Flint A., Dvorin E.L., Bischoff J. AC133-2, a novel isoform of human AC133 stem cell antigen. J. Biol. Chem. 2002, 277(23):20711-20716.
13. Mancuso P, Burlini A, Pruneri G, Goldhirsch A, Martinelli G, and Bertolini F. Resting and activated endothelial cells are increased in the peripheral blood of cancer patients. Blood 2001, 97(11):3658-3661.

## Claims

1. A method for monitoring treatment of an individual suffering from a disease comprising:
- determining whether a sample from said individual comprises AC133 mRNA in an amount that is indicative for an effective treatment, wherein said disease comprises the presence of a tumor.

2. A method according to claim 1, wherein said sample from said individual was obtained after initiation of said treatment

3. A method according to any one of claims 1-2, wherein said amount is quantified.

4. A method according to any one of claims 1-3 comprising comparing said amount with a reference value.

5. A method according to any one of claims 1-4, comprising comparing said amount of said expression product with an amount of said expression product present in a sample that was obtained from said individual before said treatment.

6. A method according to any one of claims 1-5. wherein said disease comprises the presence of a progressive tumor.

7. A method according to any one of claims 1-6 wherein said tumor comprises mouth bottom carcinoma, adenoidcystic carcinoma, renal cell carcinoma, colon carcinoma, an esophagus tumor, mesothelioma, pancreas tumor, bladder tumor, adenocarcinoma of unknown primary (ACUP), prostate tumor, renal adenocarcinoma, head and neck cancer and/or malignant melanoma.

8. A method according to any one of claims 1-7, wherein said tumor comprises breast cancer, colorectal cancer, prostate cancer and/or ovarium cancer.

9. A method according to any one of claims 1-8, wherein said tumor comprises breast cancer and/or colorectal cancer.

10. A method according to any one of claims 1-9, wherein said sample comprises a significant amount of non-endothelial cells.

11. A method according to any one of claims 1-10, wherein said sample is an essentially cell-free sample.

12. A method according to any one of claims 1-11, wherein said sample comprises a blood sample.

13. A method according to any one of claims 1-12, wherein said sample comprises a peripheral blood mononuclear cell.

14. A method according to any one of claims 1-13, wherein said treatment comprises the use of at least one of the following drugs: 2ME2, ABT510, ABT751, Angiostatin, Angiozyme, Anti-VEGF RhuMAb, Apra (CT-2584), Avicine, Benefin, BMS275291, Carboxyamidotriazole, Cisplatin, CGC4047, CC5013, CC7085, CDC801, CGP-41251 (PKC 412), CM101, Combretastatin A-4 Prodrug, DMXAA, EMD 121974, Endostatin, Enzastaurin HCl, Flavopiridol, Gemcitibine, Genistein (GCP), Green Tea Extract, IM-862, ImmTher, Interferon alpha, Interleukin-12, Iressa (ZD1839), LY317615, Marimastat, Metastat (Col-3), Neovastat, Octreotide, Paclitaxel, Penicillamine, Photofrin, Photopoint, PI-88, Prinomastat (AG-3340), PTK787 (ZK22584), RO317453, Solimastat, Squalamine, SU 101, SU11248, SU 5416, SU-6668, Suradista (FCE 26644), Suramin (Metaret). Tetrathiomolybdate, Thalidomide, TNP-470, VEGF trap, ZD6126 and/or Vitaxin.

15. A method according to any one of claims 1-14, wherein said sample is obtained within a month of initiation of said treatment.

16. A method according to any one of claims 1-15, wherein said sample is obtained within a week of initiation of said treatment.

17. A method according to any one of claims 1-16, wherein said sample is obtained within two days of initiation of said treatment.

18. A method according to any one of claims 1-17, wherein at least one primer and/or probe depicted in table 2 is used.

## Patentansprüche

1. Verfahren zur Überwachung der Behandlung eines Individuums, das an einer Krankheit leidet, umfassend die Bestimmung, ob eine Probe von dem Individuum AC133 mRNA in einer Menge umfasst, die eine effektive Behandlung anzeigt, wobei die Krankheit die Anwesenheit eines Tumors umfasst.

2. Verfahren nach Anspruch 1, wobei die Probe von dem Individuum nach Beginn der Behandlung erhalten wurde.

3. Verfahren nach einem der Ansprüche 1-2, wobei die Menge quantitativ bestimmt wird.

4. Verfahren nach einem der Ansprüche 1-3, umfassend den Vergleich der Menge mit einem Referenzwert.

5. Verfahren nach einem der Ansprüche 1-4, umfassend den Vergleich der Menge des Expressionsprodukts mit einer Menge des Expressionsprodukts, das in der Probe vorhanden ist, die von dem Individuum vor der Behandlung erhalten wurde.

6. Verfahren nach einem der Ansprüche 1-5, wobei die Krankheit die Anwesenheit eines progressiven Tumors umfasst.

7. Verfahren nach einem der Ansprüche 1-6, wobei der Tumor basales Mundkarzinom, adenoidzystisches Karzinom, Nierenzellkarzinom, Kolonkarzinom, Ösophagustumor, Mesotheliom, Pankreastumor, Blasentumor, Adenokarzinom unbekannter primärer Genese (ACUP), Prostatatumor, renales Adenokarzinom, Kopf- und Nackenkrebs und/oder malignes Melanom umfasst.

8. Verfahren nach einem der Ansprüche 1-7, wobei der Tumor Brustkrebs, Kolorektalkrebs, Prostatakrebs und/oder Eierstockkrebs umfasst.

9. Verfahren nach einem der Ansprüche 1-8, wobei der Tumor Brustkrebs und/oder Kolorektalkrebs umfasst.

10. Verfahren nach einem der Ansprüche 1-9, wobei die Probe eine signifikante Menge an Nicht-Endothelzellen umfasst.

11. Verfahren nach einem der Ansprüche 1-10, wobei die Probe eine im Wesentlichen zellfreie Probe ist.

12. Verfahren nach einem der Ansprüche 1-11, wobei die Probe eine Blutprobe umfasst.

13. Verfahren nach einem der Ansprüche 1-12, wobei die Probe eine periphere mononukleäre Blutzelle umfasst.

14. Verfahren nach einem der Ansprüche 1-13, wobei die Behandlung die Verwendung mindestens eines der folgenden Arzneimittel 2ME2, ABT510, ABT751, Angiostatin, Angiozyme, Anti-VEGF RhuMAb, Apra (CTG-2548), Avicine, Benefin, BMS275291, Carboxyamidotriazol, Cisplatin, CC4047, CC5013, CC7085, CDC801, CGP-41251 (PKC 412), CM101, Combretastatin A-4 Prodrug, DMXAA, EMD 121974, Endostatin, Enzastaurin HCl, Flavopiridol, Gemcitibine, Genistein (GCP), Grünteeextrakt, IM-862, ImmTher, Interferon alpha, Interleukin-12, Iressa (ZD1839), LY317615, Marimastat, Metastat (Col-3), Neovastat, Octreotid, Paclitaxel, Penicillamin, Photofrin, Photopoint, PI-88, Prinomastat (AG-3340), PTK787 (ZK22584), R0317453, Solimastat, Squalamine, SU 101, SU11248, SU 5416, SU-6668, Suradista (FCE 26644), Suramin (Metaret), Tetrathiomolybdat, Thalidomid, TNP-470, VEGF trap, ZD6126 und/oder Vitaxin umfasst.

15. Verfahren nach einem der Ansprüche 1-14, wobei die Probe innerhalb eines Monats nach Beginn der Behandlung erhalten wird.

16. Verfahren nach einem der Ansprüche 1-15, wobei die Probe innerhalb einer Woche nach Beginn der Behandlung erhalten wird.

17. Verfahren nach einem der Ansprüche 1-16, wobei die Probe innerhalb von zwei Tagen nach Beginn der Behandlung erhalten wird.

18. Verfahren nach einem der Ansprüche 1-17, wobei mindestens ein Primer und/oder eine Sonde gemäß Tabelle 2 verwendet wird.

## Revendications

1. Un procédé pour le monitorage du traitement d'un patient souffrant d'un affection comprenant:
- la détermination sur un échantillon prélevé sur ledit patient de la présence de mRNA AC 133 en une quantité qui est significative pour nécessiter un traitement efficace, ladite affection impliquant la présence d'une tumeur ;

2. Un procédé selon la revendication 1, dans lequel ledit échantillon prélevé sur ledit patient est obtenu après que ledit traitement a été commencé.

3. Un procédé selon l'une quelconque des revendications 1-2, dans lequel ladite quantité a été quantifiée.

4. Un procédé selon l'une quelconque des revendications 1-3 comprenant la comparaison de ladite quantité avec une valeur de référence.

5. Un procédé selon l'une quelconque des revendications 1-4, comprenant la comparaison de ladite quantité dudit produit d'expression avec une quantité dudit produit d'expression présent dans un échantillon qui a été prélevé sur ledit patient avant ledit traitement.

6. Un procédé selon l'une quelconque des revendications 1-5, dans lequel ladite affection implique la présence d'une tumeur progressive.

7. Un procédé selon l'une quelconque des revendications 1-6, dans lequel ladite tumeur consiste en carcinome du fond de bouche, carcinome adénoidcystique, carcinome des cellules rénales, carcinome du colon, tumeur de l'oesophage, mésothélium, tumeur du pancréas, tumeur de la vessie, adénocarcinomes primitifs inconnu (ACUP), tumeur de la prostate, adénocarcinome rénal, cancer de la tête et du cou et/ou mélanome malin

8. Un procédé selon l'une quelconque des revendications 1-7, dans lequel ladite tumeur consiste en cancer du sein, cancer colorectal, cancer de la prostate et/ou cancer ovarien.

9. Un procédé selon l'une quelconque des revendications 1-8, dans lequel ladite tumeur consiste en cancer du sein et/ou cancer colorectal.

10. Un procédé selon l'une quelconque des revendications 1-9, dans lequel ledit échantillon comprend une quantité significative de cellules non-endothéliales.

11. Un procédé selon l'une quelconque des revendications 1-10, dans lequel ledit échantillon est un échantillon essentiellement acellulaire.

12. Un procédé selon l'une quelconque des revendications 1-11, dans lequel ledit échantillon consiste en un échantillon de sang.

13. Un procédé selon l'une quelconque des revendications 1-12, dans lequel ledit échantillon consiste en une cellule mononucléaire de sang périphérique.

14. Un procédé selon l'une quelconque des revendications 1-13, dans lequel ledit traitement comprend l'emploi d'au moins un des médicaments suivants: 2ME2, ABT510, ABT751, Angiostatine, Angiozyme, Anti-VEGF RhuMab, Apra (CT-2584), Avicine, Bénéfine, BMS275291, Carboxyamidotriazole, Cisplatin, CC4047, CC5013, CC7085, CDC801, CGP-41251 (PKC412), CM101, Combretastatine A-4 Promédicament, DMXAA, EMD 121974, Endostatine, Enzastaurine•HCl, Flavopiridol, Gemcitibine, Genistein (GCP), Extrait de thé vert, IM-862, ImmTher, Interpheron alpha; Interleukine-12, Iressa (ZD1839), LY317615, Marimastat, Metastat (Col-3), Neovastat, Octreotide, Paclitaxel, Pénicillamine, Photofrine, Photopoint, PI-88, Prinomastat (AG-3340), PTK787 (ZK22584), R0317453, Solimastat, Squalamine, SU 101, SU11248, SU 5416, SU-6668, Suradista (FCE 26644), Suramin (Metaret), Tetrathiomolybdate, Thalidomide, TNP-470, VEGF trap, ZD6126 et/ou Vitaxin.

15. Un procédé selon l'une quelconque des revendications 1-14, dans lequel ledit échantillon est obtenu dans le mois suivant le commencement dudit traitement.

16. Un procédé selon l'une quelconque des revendications 1-15, dans lequel ledit échantillon est obtenu dans la semaine suivant le commencement dudit traitement.

17. Un procédé selon l'une quelconque des revendications 1-16, dans lequel ledit échantillon est obtenu dans les deux jours suivant le commencement dudit traitement.

18. Un procédé selon l'une quelconque des revendications 1-17, dans lequel au moins une amorce et/ou une sonde telle que décrite dans le tableau 2 est employée.
